Europäisches Patentamt

European Patent Office

Office européen des brevets

(19)

(11) Publication number: **0 138 337**
**A1**

# EUROPEAN PATENT APPLICATION

(12)

(21) Application number: 84305790.2

(22) Date of filing: 23.08.84

(51) Int. Cl.⁴: **C 12 N 15/00**
**// C12R1/29**

(30) Priority: 23.08.83 JP 154526/83

(43) Date of publication of application: 24.04.85
Bulletin 85/17

(84) Designated Contracting States: DE FR GB

(71) Applicant: **Tanabe Seiyaku Co., Ltd.,
No. 21 Dosho-machi 3-chome Higashi-ku, Osaka-shi
Osaka-fu (JP)**

(72) Inventor: **Takeda, Katsuo, No. 10-20,
Komagome 4-chome, Toshima-ku Tokyo-to (JP)**
Inventor: **Oshida, Tadahiro, No. 7-5-102,
Sakura-cho 4-chome, Hatogaya-shi Saitama-ken (JP)**
Inventor: **Yamaguchi, Totaro, No. 4-7, Ryoke 7-chome,
Urawa-shi Saitama-ken (JP)**
Inventor: **Ito, Yukio, No. 4-8-605, Ohashi 2-chome,
Meguro-ku Tokyo-to (JP)**

(74) Representative: **Bizley, Richard Edward et al, BOULT,
WADE & TENNANT 27 Furnival Street, London EC4A 1PQ
(GB)**

(54) Plasmids and their preparation and use, and protoplast-regenerated microorganisms containing such plasmids.

(57) The invention provides novel plasmids pMZ 1 and pMR 2 obtainable from a microorganism or genus Micromonospora and a method for the preparation of the same by culturing a microorganism of the genus Micromonospora containing plasmid pMZ 1 or pMR 2 in a medium, lysing the cells of the culture, and isolating plasmid pMZ 1 or pMR 2 from the lyed cells. The plasmids pMZ 1 and pMR 2 are useful as vectors in an axtinomycete host – vector system e.g. in the production of various physiologically active substances such as antibiotics from microorganisms of the genus Micromonospora and other streptomycetes by genetic engineering.

ACTORUM AG

## PLASMIDS AND THEIR PREPARATION AND USE, AND PROTOPLAST-REGENERATED MICROORGANISMS CONTAINING SUCH PLASMIDS

The present invention relates to novel plasmids and a method for the preparation thereof. More particularly, it relates to novel plasmids pMZ 1 and pMR 2 obtainable from microorganisms of the genus Micromonospora and to a method for the preparation of the same.

It is well known in the Escherichia coli host - vector system that an extrachromosomal gene plasmid is useful as a vector in genetic engineering. Recently, active studies on genetic engineering techniques have been made with a view to applying such techniques to the actinomycetes which are capable of producing various physiologically active substances such as antibiotics. However, since the useful actinomycetes-produced products usually require sets of several enzymes for their biosynthesis, it is not easy to transfer all of the corresponding genes to Escherichia coli and to express the genes within the cells thereof. It has therefore been desired to obtain a vector for which actinomycetes microorganisms can be used as a host. Various attempts have been made to use some plasmids isolated from Actinomycetes. However, almost all of such attempts have been on microorganisms of the genus Streptomycetes [e.g., Molecular and General Genetics, 185 (1982), 223-238], and with respect to the genus Micromonospora only plasmid pATM 2 is known, which is obtained from Micromonospora coerulea ATCC 27008 (cf. Japanese Patent First Publication No. 179196/1982).

The present invention is based upon the discovery, isolation and characterization of novel plasmids from antibiotic-producing strains of the genus Micromonospora, i.e. aminoglycoside antibiotics-producing strains and macrolide antibiotics-producing strains.

The present invention provides a novel plasmid pMZ 1 which has a molecular weight of 6.4 ± 0.2 megadaltons and has the following number of sites sensitive to restriction enzymes:

Kpn I: 1, Bam HI: 1, Bgl II: 2, Bcl I: 2, Hpa I: 2, Eco RI: 0, Hind III: 0, Pvu II: 0, Xho I: 0

and a novel plasmid pMR 2 which has a molecular weight of 7.4 ± 0.2 megadaltons and has the following number of sites sensitive to restriction enzymes:

Hind III: 1, Eco RI: 1, Bcl I: 1, Pvu II: 1, Sma I: 2, Bam HI: 3, Hpa I: 0, Xho I: 0, Pst I: 0.

These plasmids can be prepared by cultivating a microorganism of the genus <u>Micromonospora</u> containing the plasmid pMZ 1 or pMR 2 in a medium, lysing the cells of the culture, and isolating the plasmid pMZ 1 or pMR 2 from the lysed cells.

Plasmid pMZ 1 is a deoxyribonucleic acid having a molecular weight of 6.4 ± 0.2 megadaltons and has the following number of sites sensitive to restriction enzymes:

| Restriction enzymes | Number of cleavage sites |
|---|---|
| <u>Kpn</u> I | 1 |
| <u>Bam</u> HI | 1 |
| <u>Bgl</u> II | 2 |
| <u>Bcl</u> I | 2 |
| <u>Hpa</u> I | 2 |
| <u>Eco</u> RI | 0 |
| <u>Hind</u> III | 0 |
| <u>Pvu</u> II | 0 |
| <u>Xho</u> I | 0 |

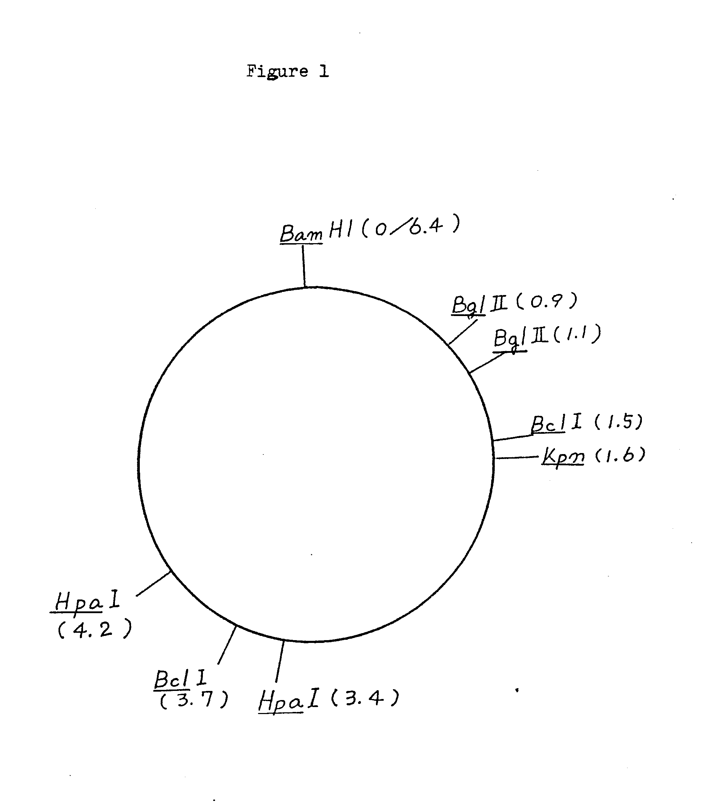

and has a restriction enzyme map as shown in the accompanying Fig. 1.

Plasmid pMR 2 is a deoxyribonucleic acid having a molecular weight of 7.4 ± 0.2 megadaltons and has the following number of sites sensitive to restriction enzymes:

| Restriction enzymes | Number of cleavage sites |
|---|---|
| <u>Hind</u> III | 1 |
| <u>Eco</u> RI | 1 |
| <u>Bcl</u> I | 1 |
| <u>Pvu</u> II | 1 |
| <u>Sma</u> I | 2 |
| <u>Bam</u> HI | 3 |
| <u>Hpa</u> I | 0 |
| <u>Xho</u> I | 0 |
| <u>Pst</u> I | 0 |

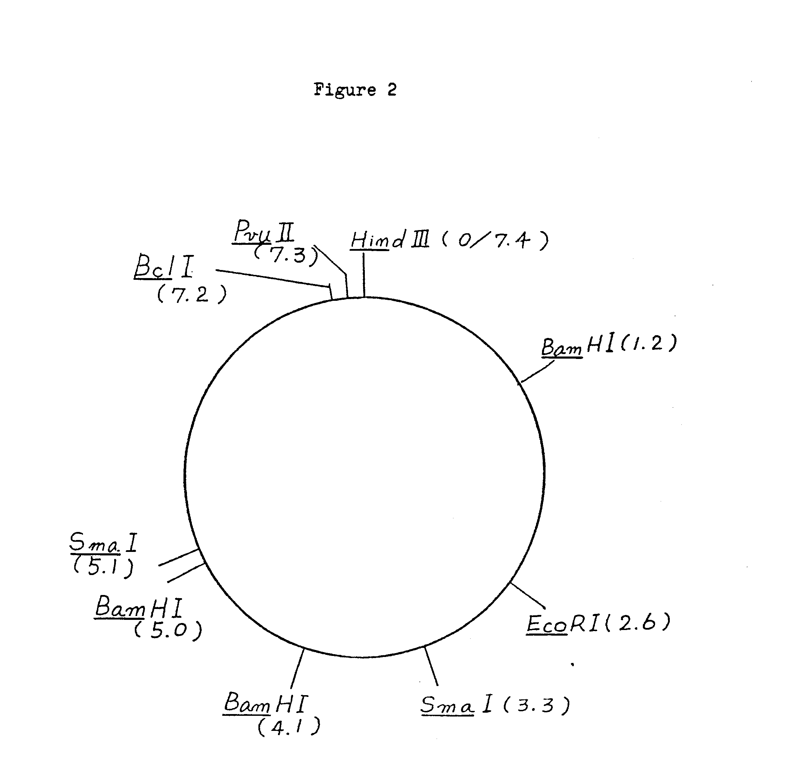

and has a restriction emzyme map as shown in the accompanying Fig. 2.

The above-mentioned restriction enzymes are referred to using abbreviations derived from the microorganisms from which they originate, as follows:

| | |
|---|---|
| <u>Bam</u> HI: | <u>Bacillus</u> <u>amyloliquefaciens</u> H |
| <u>Bgl</u> II: | <u>Bacillus</u> <u>globigii</u> |
| <u>Kpn</u> I: | <u>Klebsiella</u> <u>pneumoniae</u> |
| <u>Hpa</u> I: | <u>Haemophilus</u> <u>parainfluenzae</u> |
| <u>Hind</u> III: | <u>Haemophilus</u> <u>influenzae</u> |
| <u>Eco</u> RI: | <u>Escherichia</u> <u>coli</u> PY13 (R1) |
| <u>Sma</u> I: | <u>Serratia</u> <u>marcescens</u> Sb |
| <u>Bcl</u> I: | <u>Bacillus</u> <u>caldolyticus</u> |
| <u>Pvu</u> II: | <u>Proteus</u> <u>vulgaris</u> |
| <u>Xho</u> I: | <u>Xanthomonas</u> <u>holcicola</u> |
| <u>Pst</u> I: | <u>Providencia</u> <u>stuartii</u> |

These restriction ensymes are commercially available from Takara Shuzo K.K. (Japan) and New England Biolabs. Co. (U.S.A.).

Microorganisms useful for the preparation of the plasmids of the present invention include all microorganisms of the genus Micromonospora containing the plasmid pMZ 1 or pMR 2, for example, Micromonospora zionensis or Micromonospora rosaria, more specifically, Micromonospora zionensis NRRL 5466 or Micromonospora rosari NRRL 3718, which are available from Northern Regional Research Laboratory, U.S.A. The morphological characteristics of Micromonospora zionensis NRRL 5466 and Micromonospora rosaria NRRL 3718 are described in The Journal of Antibiotics, 29, 483-487 (1976) and ibid., 25, 641-646 (1972), respectively.

Media useful for cultivating these microorganisms include any conventional medium wherein the microorganisms can grow retaining the plasmids within the cells. An example of the medium contains carbon sources such as e.g. glucose, sucrose, molasses, organic acids, dextrin or starches, nitrogen sources such as e.g. peptone, meat extract, bact.yeast extract (manufactured by Difco, U.S.A.), Kazamino Acid (manufactured by Difco, U.S.A.) or NZ-amine (manufactured by Shefield, U.S.A.), and various inorganic salts such as e.g. phosphates, calcium salts,

- 6 -                          0138337

magnesium salts, potassium salts, sodium salts, manganese salts and iron salts.  The medium may also contain e.g. vitamins and various amino acids, depending upon the microorganism strain used.

The culture of the microorganisms can be carried out by conventional methods, for example, at a temperature of about 20 to $32^{\circ}C$, preferably about 27 to $32^{\circ}C$, for a period of time of about 3 to 10 days, preferably about 4 to 7 days.

In order to obtain the desired plasmids in good efficiency, it is preferable to form protoplasts from the <u>Micromonospora</u> microorganism, to culture the protoplasts in a regeneration medium to obtain cells regenerated from the protoplasts, and then to culture the regenerated cells in a medium under the above conditions.  Suitable examples of protoplast-regenerated strains are <u>Micromonospora</u> <u>zionensis</u> MCRL 6001 (deposition number at the Fermentation Research Institute, Agency of Industrial Science and Technology, Japan, under Budapest treaty: FERM BP-563) and <u>Micromonospora</u> <u>rosaria</u> MCRL 6101 (FERM BP-564).  Both of these strains were deposited at the Fermentation Research Institute on 11th July 1983. The morphological characteristics of <u>Micromonospora</u> <u>zionensis</u> MCRL 6001 (FERM BP-563) are the same as those of the parent strain (i.e., <u>Micromonspora</u> <u>zionensis</u> NRRL 5466) except that the former strain has only one kind of plasmid

(i.e., pMZ l) whereas the latter strain has two kinds of plasmids. The morphological characteristics of Micromonospora rosaria MCRL 6101 (FERM BP-564) are the same as those of the parent strain (i.e., Micromonospora rosaria NRRL 3718) except that the former strain has only one kind of plasmid (i.e., pMR 2) whereas the latter strain has two kinds of plasmids.

The formation and regeneration of such protoplasts can be carried out by a known method as used, for example, for streptomycete protoplasts [cf. Journal of General Microbiology, 80, 389 - 400 (1974)]. In this method for formation of protoplasts, the buffer solution used, however, preferably has a comparatively lower concentration of salts than usual, e.g. a concentration of 1 mM to 10 mM of salts such as magnesium, calcium, potassium and sodium salts. Also, the medium used for the regeneration of protoplasts preferably has a comparatively lower concentration of sucrose than usual, e.g. a concentration of O.2 to O.3 M of sucrose. The protoplast-regenerated strain can be obtained, for example, by cultivating the starting microorganism in a medium having a concentration of glycine (e.g. O.05 % to O.3 %) at which the growth of the microorganism is slightly inhibited, collecting the cells, lysing the cell walls of the cells with a cell-lytic enzyme such as lysozyme in a buffer for the formation of protoplasts which contains e.g. sucrose, $Mg^{2+}$ and $Ca^{2+}$ to form protoplasts, and then cultivating the protoplasts thus-formed in a medium for regeneration of protoplasts which contains sucrose at a concentration of 0.2 to 0.3 M.

The cell-lysis can be performed by using any conventional method, for example, using a cell-lytic enzyme such as lysozyme, and various other enzymes can also be used [e.g. Pronase E (manufactured by Kaken Yakuhin K.K., Japan) or Ribonulease A manufactured by Miles Laboratories, South Africa)] for the purpose of eliminating proteins and ribonucleic acids, and various surfactants [e.g. SDS (manufactured by Wako Junyaku K.K., Japan) or sarcosin] for the purpose of promoting the cell-lysis.

Isolation of the plasmids from the lysed cells can be carried out by known methods, for example, by the ethanol precipitation method, sucrose density gradient method, cesium chloride-ethidium bromide equilibrium density gradient centrifugation method, dialysis, or by a combination of two or more of such methods.

The thus-obtained plasmids pMZ 1 and pMR 2 are autonomously replicable in Micromonospora zionensis, Micromonospora rosaria, or industrially useful homologous microorganisms, and have cleavage sites for various restriction enzymes, and hence they are useful as vectors

- 9 -

0138337

for recombining genes which participate in the production of e.g. various useful physiological substances such as antibiotics from microorganisms of the genus _Micromonospora_ and other steptomycetes.

Recombination of a gene with the plasmids of the present invention to obtain a recombinant plasmid can be carried out by the conventional methods [cf. Scientific American, 233, 1, 24-33 (1975)], and further, transformation of a host microorganism with the recombinant plasmid can also be carried out by conventional methods [cf. Nature, 274, 398-400 (1978), Journal of Bacteriology, 151, 668-677 (1982)].

The present invention is illustrated by the following Example, but should not be construed to be limited thereto. In the Example, "%" means w/v % unless specified otherwise.

Example

Preparation of plasmid pMZ 1:

(1)   Preparation of protoplast

_Micromonospora_ _zionensis_ NRRL 5466 strain is inoculated into a 250 ml flask containing 50 ml of S medium [glucose 1.0 %, polypeptone 0.3 %, bact. yeast extract 0.4 %, magnesium sulfate heptahydrate 0.05 %, dipotassium hydrogen phosphate 0.2 %, disodium hydrogen phosphate dodecahydrate 0.8 %] and shake-cultured at 32°C for 6 days.

- 10 -

0138337

The culture broth (8 ml) is transferred to a 500 ml flask containing 100 ml of S medium containing additionally glycine 0.1 %, and then shake-cultured at 32°C for 18 hours. The culture broth (8 ml) is centrifuged (8,000 rpm, for 10 minutes, at 4°C) to collect the cells. The cells are washed with 17.1 % sucrose (8 ml), and then again centrifuged likewise to collect the cells. The cells are suspended in M1 buffer [25 mM Tris-HCl (pH 7.1), 0.5 M sucrose, 5 mM magnesium chloride hexahydrate, 5 mM calcium chloride dihydrate, 5 mM sodium chloride] and thereto is added lysozyme at a final concentration of 1 mg/ml, and the mixture is cultured at 30°C for 1 - 3 hours to obtain protoplasts.

(2)    Isolation of a protoplast-regenerated strain containing pMZ 1

The protoplasts thus obtained are appropriately diluted with M1 buffer. The diluted protoplast (50 $\mu$l) is spread on MR1 medium for regeneration [0.025 M DOTITE TES (i.e. N-tris(hydroxymethyl)methyl-2-aminoethanesulfonic acid, pH 7.2 with NaOH), 0.2 M sucrose, glucose 1.0 %, polypeptone 0.3 %, bact. yeast extract 0.4 %, magnesium chloride hexahydrate 0.3 %, calcium chloride dihydrate 0.02 %, L-glutamine 0.2 %, agar 1.8 %] and cultured at 30°C for 10 - 20 days to grow colonies. The colonies are transferred to MR1 agar slant medium, and further cultured at 32°C for 7 - 10 days.  Each colony thus obtained is inoculated into a test tube (inner diameter: 21 mm) containing 10 ml of 0.1 % glycine-containing GPY medium [glucose 1.0 %, polypeptone 0.3 %, bact. yeast extract 0.1 %, NaCl 0.4 %, magnesium sulfate heptahydrate 0.04 %, dipotassium hydrogen phosphate 0.2 %, disodium hydrogen phosphate dodecahydrate 0.8 %] and then subjected to reciprocal shake-culture at 28°C for 5 - 10 days.  The culture broth (1 ml) is placed in a 1.5 ml test tube (manufactured by Eppendorf, Co.) and centrifuged (12,000 rpm, for 5 minutes, at room temperature) to collect the cells.

The cells are suspended in 300 $\mu$l of lysozyme solution [which is prepared by dissolving lysozyme (2 mg/ml) in 0.05 M TES buffer (0.05 M Trisaminomethane-HCl (pH 7.4), 0.05 M ethylenediamine tetraacetate (EDTA), 0.05 M sodium chloride)] and then reacted at 37°C for 20 - 30 minutes.  To the reaction mixture is added 300 $\mu$l of a cell-lytic solution [0.5 M sodium chloride, 2 % SDS, 0.0625 M cyclohexanediamine tetraacetate] and the mixture is reacted at 37°C for 15 minutes to lyse the cells.  To the reaction mixture is added 600 $\mu$l of a phenol-chloroform solution [a lower layer of a solution prepared by mixing phenol 500 g, chloroform 500 ml, 0.5 N sodium hydroxide 200 ml, and 8-hydroxyquinoline 0.5 g], and the mixture is stirred with a vortex mixer for 30 - 60 seconds and centrifuged (12,000 rpm, for 5 minutes, at room temperature). The upper layer (30 - 50 $\mu$l) of the mixture thus obtained is subjected to agarose gel electrophoresis to detect plasmids, by which a strain having only pMZ 1 is selectively collected from the protoplast-regenerated strains to give the desired protoplast-regenerated strain MCRL 6001 (FERM BP-563).

(3)    Cultivation of the protoplast-regenerated strain

_Micromonospora_ _zionensis_ MCRL 6001 thus obtained is inoculated into a 250 ml flask containing 50 ml of S medium, and shake-cultured at 32°C for 6 days.  The culture broth (8 ml) is transferred to a 500 ml flask containing 100 ml of a 0.1 % glycine-containing GPY medium, and subjected to rotary culture at 32°C for 1 - 2 days.  The culture broth is centrifuged (5,000 rpm, for 10 minutes, at 4°C) to collect the cells.  The cells are suspended in 0.05 M TES buffer, and then again centrifuged (10,000 rpm, for 20 minutes, at 4°C) to give washed cells.

(4)    Isolation of plasmid pMZ 1

The cells obtained above are again suspended in 0.05 M TES buffer at a concentration of 1 g of the wet cells per 20 ml of the buffer, and thereto are added lysozyme (a 4 % solution in 0.05 M TES buffer) at a final concentration of 1 mg/ml and ribonuclease A (manufactured by Miles Laboratories, a solution in 0.8 % NaCl so as to be in a concentration of 5 mg/ml, which is previously heat-treated at 95°C for 10 minutes) at a final concentration of 50 $\mu$g/ml. The mixture is reacted at 37°C for one hour, and to the reaction mixture is added 10 % SDS (dissolved in 0.05 M TES buffer) in a final concentration of 1.5 %, and the mixture is reacted at 37°C for 20 minutes to completely lyse the cells. The cell-lysed solution is centrifuged (20,000 rpm, for 20 minutes, at 20°C) and the supernantant is collected and thereto is added 5 M NaCl (dissolved in 0.05 M TES buffer) at a final concentration of 1 M, and the mixture is stirred. Thereafter, the mixture is ice-cooled for 2 hours and centrifuged (6,000 rpm, for 15 minutes, at 0°C) to collect the suppernatant. To the supernatant is added Pronase E (a 5 mg/ml solution in 0.05 M TES buffer) at a final concentration of 100 $\mu$g/ml, and the mixture is reacted at 37°C for one hour. To the reaction mixture is added 40 % polyethylene glycol 6,000 (dissolved in 0.05 M TES buffer) at a final concentration of 10 %, and the mixture is well stirred and allowed to stand at 4°C overnight. The mixture is centrifuged (3,500 rpm, for 10 minutes, at 0°C) to collect precipitates of plasmid DNA. The plasmid DNA precipitates thus obtained are dissolved in 0.025 M TES buffer (3 ml), and the solution is dialysed against the same buffer at room temperature for more than 3 hours. To the dialysed solution is added 0.025 M TES buffer so as to total 4 ml, and therein is dissolved cesium chloride (4.26 g). A solution (0.5 ml) of ethidium chloride (4.8 mg/ml) in 0.05 M TES buffer is uniformly mixed with the above reaction mixture. The resulting mixture is transferred into a Quick-seal polyallomer tube (manufactured by Beckmann) and after heat-sealing the tube, the mixture is subjected to equilibrium density gradient centrifugation at 60,000 rpm, 20°C overnight with a vertical rotor (VTi type 80, manufactured by Beckmann). A fraction (about 0.5 ml) of the plasmid band, confirmed under ultraviolet (320 nm), is collected from the bottom of the tube. To the fraction is added an isopropyl alcohol saturated with cesium chloride solution (the upper layer of a mixture prepared by mixing cesium chloride 10 g, 0.025 M TES buffer 9.4 ml and isopropyl alcohol 10 ml, followed by allowing to stand) in order to remove ethidium bromide. The plasmid-containing solution is dialysed against 0.025 M TES buffer (one time) and against DSB solution [10 mM Tris-HCl buffer (pH 7.6), 10 mM NaCl, and 1 mM EDTA] (three times). The desired pMZ 1 (30.5 $\mu$g) is obtained from the dialysed solution obtained above.

(5) Properties of plasmid pMZ 1

The molecular weight and restriction enzyme map of pMZ 1 are determined by subjecting a product digested by restriction enzymes to agarose gel electrophoresis.

The plasmid pMZ 1 (0.1 - 1.0 $\mu$g) is reacted with twice the amount of various commercially available

restriction enzymes in an appropriate solution (40 $\mu$l) at 37°C for one hour. The reaction mixture is treated with 10 $\mu$l of a reaction halting solution (50 % sucrose, 108 mM EDTA, 0.025 % bromophenol blue) at 70°C for 5 minutes, and then is rapidly cooled. The resulting solution is subjected to electrophoresis with 0.6 - 1.0 % agarose at 20 mA for 16 - 18 hours using TEAS buffer [50 mM Tris-HCl buffer (pH 8.05), 20 mM sodium acetate, 2 mM EDTA, 18 mM NaCl] as a buffer for electrophoresis. After the electrophoresis, the gel is dyed by soaking it in an ethidium bromide solution (1 $\mu$g/ml), and then DNA fragments are detected by photographing the bands which are fluorescent under ultraviolet irradiation (305 nm).

The molecular weight of each fragment is calculated by comparing with the migration distance of standard DNA fragments having known molecular weight, i.e. Hind III digested lambda DNA. The calculation of the molecular weight of each fragment is carried out in the light of value disclosed in the catalogue of New England Biolabs (1982 - 1983).

The molecular weight and restriction enzyme map of pMZ 1 are determined from the molecular weight of the fragments obtained by the treatment with one or two of the various restriction enzymes.

As a result, the plasmid pMZ 1 has a molecular weight of 6.4 ± 0.2 megadaltons, the following number of sites sensitive to restriction enzymes and a restriction enzyme map as shown in Fig. 1.

| Restriction enzymes | Number of cleavage sites |
|---------------------|--------------------------|
| Kpn I | 1 |
| Bam HI | 1 |
| Bgl II | 2 |
| Bcl I | 2 |
| Hpa I | 2 |
| Eco RI | 0 |
| Hind III | 0 |
| Pvu II | 0 |
| Xho I | 0 |

Example 2

Micromonospora rosaria NRRL 3718 strain is treated in the same manner as described above to give a protoplast-regenerated strain, and the protoplast-regenerated strain is cultured likewise and the presence of plasmids is checked in the colonies thus produced in the same manner as described above. The strain having only pMR 2 is collected to give a pure protoplast-regenerated strain, Micromonospora rosaria MCRL 6101 (FERM BP-564). The strain is cultured, cell-lysed in the same manner as described above, and the plasmid DNA is isolated to give pMR 2 (49 $\mu$g) (from the culture broth 600 ml).

The molecular weight and restriction enzyme map of the pMR 2 are determined in the same manner as described above.

As a result, the plasmid pMR 2 has a molecular weight of 7.4 ± 0.2 megadaltons, the following number of

0138337

sites sensitive to restriction enzymes and a restriction enzyme map as shown in Fig. 2.

| Restriction enzymes | Number of cleavage sites |
|---|:---:|
| Hind III | 1 |
| Eco RI | 1 |
| Bcl I | 1 |
| Pvu II | 1 |
| Sma I | 2 |
| Bam HI | 3 |
| Hpa I | 0 |
| Xho I | 0 |
| Pst I | 0 |

0138337

CLAIMS :

1.      A plasmid designated pMZ 1 which has a molecular weight of 6.4 ± 0.2 megadaltons and has the following number of sites sensitive to restriction enzymes:

Kpn I: 1, Bam HI: 1, Bgl II: 2, Bcl I: 2, Hpa 1: 2, Eco RI: 0, Hind III: 0, Pvu II: 0, Xho I: 0

2.      A plasmid according to claim 1, which has the following restriction enzyme map:

Bam HI (0/6.4)

Bgl II (0.9)

Bgl II (1.1)

Bcl I (1.5)

Kpn (1.6)

Hpa 1 (4.2)

Bcl 1 (3.7)

Hpa I (3.4)

3.    A plasmid designated pMR 2 which has a molecular weight of 7.4 ± O.2 megadaltons and has the following number of sites sensitive to restriction enzymes:

Hind III: Eco RI: 1, Bcl I: 1, Pvu II: 1, Sma I:2
Bam HI: 3, Hpa I: O, Xho I: O, Pst I: O.

4.    A plasmid according to claim 2, which has the following restriction enzyme map:

5.    A method for preparing a plasmid pMZ 1 or pMR 2, which comprises culturing a microorganism of the genus Micromonospora containing plasmid pMZ 1 or plasmid pMR 2 in a medium, lysing the cells of the culture, and isolating plasmid pMZ 1 or pMR 2 from the lysed cells.

6.    A method according to claim 5, wherein the microorganism of the genus _Micromonospora_ is _Micromonospora_ _zionensis_ NRRL 5466 or _Micromonospora_ _rosaria_ NRRL 3718.

7.    A method according to claim 5, wherein the microorganism of the genus _Micromonospora_ is protoplast-regenerated strain _Micromonospora_ _zionensis_ MCRL 6001 (FERM BP-563) or protoplast-regenerated strain _Micromonospora_ _rosaria_ MCRL 6101 (FERM BP-564).

8.    A protoplast-regenerated strain of the genus _Micromonospora_ containing plasmid pMZ 1 or plasmid pMR 2.

9.    A strain as claimed in claim 8 which is _Micromonospora_ _rosaria_ NRRL 3718 or _Micromonospora_ _rosaria_ MCRL 6101 (FERM BP-564).

10.    The use of plasmid pMZ 1 or plasmid pMR 2 as a vector in a genetic engineering technique for the production of a desired substance, e.g. an antibiotic.

Figure 1

Figure 2

European Patent
Office

**EUROPEAN SEARCH REPORT**

. Application number

| | **DOCUMENTS CONSIDERED TO BE RELEVANT** | | | EP 84305790.2 |
|---|---|---|---|---|
| Category | Citation of document with indication, where appropriate, of relevant passages | | Relevant to claim | CLASSIFICATION OF THE APPLICATION (Int. Cl.4) |
| D,A | EP - A1 - 0 064 350 (TAKEDA CHEMICAL INDUSTRIES, LTD.)<br>* Claims 1-7,9 *<br>& JP-A2-179 196/1982<br>---- | | 1-5 | C 12 N 15/00<br>//C 12 R 1:29 |
| | | | | **TECHNICAL FIELDS SEARCHED (Int. Cl.4)**<br><br>C 12 N |

The present search report has been drawn up for all claims

| Place of search | Date of completion of the search | Examiner |
|---|---|---|
| VIENNA | 17-12-1984 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82